# EUROPEAN PATENT APPLICATION

(11) **EP 3 861 993 A1**
(43) Date of publication of application: **11.08.2021**
(21) Application number: 19868694.1
(22) Date of filing: 04.10.2019
(51) Int. Cl.: A61K 31/197, A61K 9/08, A61K 9/70, A61K 47/04, A61K 47/10, A61K 47/12, A61K 47/20, A61P 25/08

(54) **PREPARATION STABILIZED BY MEANS OF NONAQUEOUS SOLVENT**

(30) Priority: 04.10.2018 JP 2018189491; 30.01.2019 JP 2019014911
(71) Applicant: MEDRx Co., Ltd., Higashikagawa-shi Kagawa 769-2712 (JP)
(72) Inventor: NAKAMURA, Jun, Higashikagawa-shi, Kagawa 769-2712 (JP); MIWA, Yasushi, Higashikagawa-shi, Kagawa 769-2712 (JP); YAMASAKI, Keiko, Higashikagawa-shi, Kagawa 769-2712 (JP); HAMAMOTO, Hidetoshi, Higashikagawa-shi, Kagawa 769-2712 (JP)
(74) Representative: Vossius & Partner Patentanwälte Rechtsanwälte mbB
(86) International application number: PCT/JP2019/039349
(87) International publication number: WO 2020/071546

(57) **Abstract**

The present invention provides a composition for external application comprising baclofen or a salt thereof, specifically baclofen hydrochloride with high transdermal absorbability and stability, and a method of stabilizing a preparation comprising baclofen or a salt thereof.

## Description

### TECHNICAL FIELD

The present invention relates to a composition for external application comprising baclofen or a salt thereof and a method of stabilizing a preparation comprising baclofen or a salt thereof.

### BACKGROUND ART

Baclofen has been commercially available as oral tablets and intrathecal injections, and has been used as an agent for treating a disease such as spastic paralysis. Transdermal administration is desirable as an administration method of keeping the effective concentration in plasma at a certain level without causing sides effects on the digestive system such as nausea caused by the administration of oral preparations. As an external preparation comprising baclofen, an aqueous preparation comprising an alcohol of 6 to 12 carbon atoms and propylene glycol has been suggested (Patent Document 1).

Baclofen is easily dehydrated and condensed. Hence, it is necessary to consider inhibiting the deterioration of baclofen over time when preparing a preparation comprising baclofen. As a method of inhibiting the deterioration of baclofen over time, a method of adding α-amino acid has been suggested (Patent Document 2).

### PRIOR ART DOCUMENTS

### PATENT DOCUMENTS

Patent Document 1: JP S63-253022
Patent Document 2: JP 2000-34226

### SUMMARY OF THE INVENTION

### (Problems to be solved by the invention)

An object of the present invention is to provide a composition for external application comprising baclofen or a salt thereof, specifically baclofen hydrochloride with high transdermal absorbability and stability.

Also, an object of the present invention is to provide a preparation comprising baclofen or a salt thereof with high stability, which inhibits the cyclocondensation reaction of baclofen.

### (Means for Solving the Problems)

The present inventors have extensively studied to reach the above objects, and have found that a specific acid can function as a solubilizing agent, a stabilizing agent, and/or a transdermal absorption enhancer of baclofen. In addition, the present inventors have found that when a salt of baclofen with the specific acid is dissolved in dimethyl sulfoxide (hereinafter, also referred to as "DMSO"), the generation of ring products (intramolecular condensation products) and other degradation products of baclofen is inhibited, and thus high storage stability of baclofen can be produced. Based upon the new findings, the present invention has been completed.

That is, the present invention provides the following items [1] to [4].
[1] An external preparation comprising baclofen hydrochloride.
[2] The external preparation according to the above item [1], which further comprises DMSO.
[3] A method of stabilizing a preparation comprising baclofen, which comprises dissolving baclofen hydrochloride in an organic solvent or an inorganic solvent.
[4] An external preparation comprising baclofen hydrochloride, DMSO and a C₁₆₋₂₀ fatty acid.

In addition, the present invention provides the following items [5] to [12].
[5] A composition for external application comprising baclofen hydrochloride.
[6] The composition for external application according to the above item [5], which further comprises DMSO.
[7] The composition for external application according to the above item [5] or [6], which further comprises one or more C₁₆₋₂₀ fatty acids.
[8] The composition for external application according to the above item [7], wherein the C₁₆₋₂₀ fatty acid is oleic acid, isostearic acid or a mixture thereof.
[9] A patch preparation comprising a support and an adhesive layer laminated on one side of the support, wherein the adhesive layer comprises the composition for external application according to any one of the above items [5] to [8] .
[10] A patch preparation comprising:
   (a) a solvent-impermeable first sheet;
   (b) a solvent-impermeable second sheet attached to an upper surface of the first sheet, forming a non-sealing region and a sealing region surrounding the non-sealing region with the first sheet, and having a cutting part formed to annularly extend along an outer circumferential edge of the non-sealing region;
   (c) a transdermal absorption preparation carrying member carrying the composition for external application according to any one of the above items [5] to [8] disposed between the first sheet and the second sheet in the non-sealing region and fixed to the second sheet inside the cutting part; and
   (d) an adhesive third sheet attached in a removable manner to an upper surface of the second sheet.
[11] A method of stabilizing a preparation comprising baclofen or a salt thereof, which comprises dissolving baclofen hydrochloride in an organic solvent and/or an inorganic solvent.
[12] The method according to the above item [11], wherein the organic solvent comprises DMSO.

### (Effects of the Invention)

The present invention can inhibit the generation of ring products (intramolecular condensation products) and other degradation products of baclofen and produce high storage stability when an external preparation comprising baclofen as an active ingredient is prepared. In addition, the present invention can enhance the skin permeability of baclofen.

### DESCRIPTION OF EMBODIMENTS

### [Composition for external application comprising baclofen or salt thereof]

The composition for external application of the present invention comprises baclofen (4-amino-3-(4-chloropehnyl)butanoic acid) or a salt thereof, preferably baclofen hydrochloride. Baclofen may be in the forms of racemate or an optical isomer (R-baclofen or S-baclofen). Baclofen hydrochloride is usually dissolved in an organic solvent and/or an inorganic solvent. Examples of the organic solvent include fatty acids such as capric acid, lauric acid, myristic acid, palmitic acid, palmitoleic acid, margaric acid, stearic acid, isostearic acid, oleic acid, vaccenic acid, linoleic acid, arachidic acid and arachidonic acid; keto acids such as levulinic acid; monovalent alcohols such as capryl alcohol, cetyl alcohol, stearyl alcohol and oleyl alcohol; divalent alcohols such as propylene glycol, butylene glycol and polyethylene glycol; trivalent alcohols such as glycerin; fatty acid ester such as diethyl sebacate, isopropyl myristate, diisopropyl adipate, myristyl palmitate and stearyl stearate; diesters such as propylene glycol diacetate; diethylene glycol monoethyl ether; and dimethyl sulfoxide. Examples of the inorganic solvent include water. The solvents may be used alone. Also, two or more of the solvents may be used in combination.

Baclofen hydrochloride dissolved in an organic solvent and/or an inorganic solvent inhibits the generation of intramolecular condensation products (ring products) and other degradation products of baclofen and shows high storage stability.

In the composition for external application of the present invention, DMSO is used as the preferred solvent for dissolving baclofen hydrochloride. The skin permeability and storage stability of baclofen hydrochloride is enhanced by the addition of DMSO.

In certain embodiments, the composition for external application of the present invention may comprise additional organic solvent(s) with baclofen hydrochloride and DMSO. The organic solvents listed above may be used as the additional organic solvent(s). In certain embodiments, the composition for external application of the present invention comprises baclofen hydrochloride, DMSO and oleyl alcohol. In certain embodiments, the composition for external application of the present invention may comprise additional organic solvent(s) with baclofen hydrochloride, DMSO and oleyl alcohol.

In certain embodiments, the composition for external application of the present invention comprises baclofen hydrochloride, DMSO and a C₁₆₋₂₀ fatty acid. In certain embodiments, the composition for external application of the present invention comprises baclofen hydrochloride, DMSO, a C₁₆₋₂₀ fatty acid and propylene glycol diacetate. Examples of the C₁₆₋₂₀ fatty acid include C₁₆ saturated fatty acids such as palmitic acid, C₁₆ unsaturated fatty acids such as palmitoleic acid, C₁₈ saturated fatty acids such as stearic acid and isostearic acid, C₁₈ unsaturated fatty acids such as oleic acid, linoleic acid and linolenic acid, C₂₀ saturated fatty acids such as arachidic acid and C₂₀ unsaturated fatty acids such as arachidonic acid. The C₁₆₋₂₀ fatty acids may be used alone. Also, two or more of C₁₆₋₂₀ fatty acids may be used in combination. In certain embodiments, the C₁₆₋₂₀ fatty acid may be isostearic acid.

The concentration of baclofen or a salt thereof (preferably, baclofen hydrochloride) may be, for example, 0.2 to 40% by weight, 1 to 30% by weight, 3 to 20% by weight, 5 to 15% by weight, 5 to 12% by weight, 7 to 12% by weight, 10 to 45% by weight, 15 to 40% by weight, 17 to 35% by weight, 18 to 30% by weight or 20 to 30% by weight, relative to the total weight of the composition for external application.

In certain embodiments, the concentration of DMSO may be, for example, 20 to 99% by weight, 20 to 98% by weight, 25 to 95% by weight, 28 to 95% by weight, 30 to 95% by weight, 32 to 95% by weight, 35 to 95% by weight, 37 to 95% by weight, 39 to 95% by weight, 40 to 95% by weight, 41 to 95% by weight, 42 to 95% by weight, 43 to 95% by weight, 44 to 95% by weight, 45 to 95% by weight, 46 to 95% by weight, 47 to 95% by weight, 48 to 95% by weight, 49 to 95% by weight, 50 to 95% by weight, 51 to 95% by weight, 52 to 95% by weight, 53 to 95% by weight, 54 to 95% by weight, 55 to 95% by weight, 56 to 95% by weight, 57 to 95% by weight, 58 to 95% by weight, 59 to 95% by weight, 60 to 95% by weight, 61 to 95% by weight, 62 to 95% by weight, 63 to 95% by weight, 64 to 95% by weight, 65 to 95% by weight, 66 to 95% by weight, 67 to 95% by weight, 68 to 95% by weight, 69 to 95% by weight, 70 to 95% by weight, 71 to 95% by weight, 72 to 95% by weight, 73 to 95% by weight, 74 to 95% by weight, 75 to 95% by weight, 76 to 95% by weight, 77 to 95% by weight, 78 to 95% by weight, 79 to 95% by weight, 80 to 95% by weight, 81 to 95% by weight, 82 to 95% by weight, 83 to 95% by weight, 84 to 95% by weight or 85 to 95% by weight, relative to the total weight of the composition for external application.

In certain embodiments, the concentration of DMSO may be, for example, 5 to 50% by weight, 7 to 46% by weight, 10 to 46% by weight, 12 to 45% by weight, 13 to 44% by weight, 14 to 43% by weight, 15 to 42% by weight, 16 to 41% by weight, 17 to 40% by weight, 18 to 39% by weight, 19 to 38% by weight, 20 to 37% by weight, 21 to 36% by weight, 22 to 35% by weight, 23 to 34% by weight, 24 to 33% by weight, 25 to 32% by weight, 25 to 31% by weight or 25 to 30% by weight, relative to the total weight of the composition for external application.

In certain embodiments, the concentration of the C₁₆₋₂₀ fatty acid may be, for example, 0.5 to 60% by weight, 1 to 50% by weight, 5 to 45% by weight, 10 to 40% by weight, 10 to 38% by weight, 10 to 36% by weight, 12 to 34% by weight, 12 to 32% by weight, 13 to 30% by weight, 14 to 28% by weight, 15 to 25% by weight or 18 to 22% by weight, relative to the total weight of the preparation composition for external application.

The concentration of the C₁₆₋₂₀ unsaturated fatty acid may be, for example, 0.5 to 10% by weight, 0.6 to 8% by weight, 0.8 to 6% by weight, 0.8 to 4% by weight, 0.9 to 2.5% by weight, 1.0 to 2.2% by weight or 1.2 to 1.8% by weight, relative to the total weight of the composition for external application. The concentration of the C₁₆₋₂₀ saturated fatty acid may be, for example, 5 to 50% by weight, 10 to 45% by weight, 12 to 40% by weight, 15 to 35% by weight, 15 to 34% by weight, 15 to 33% by weight, 15 to 32% by weight, 15 to 31% by weight, 15 to 30% by weight, 15 to 29% by weight, 15 to 28% by weight, 15 to 27% by weight, 15 to 26% by weight, 15 to 25% by weight, 15 to 24% by weight, 15 to 23% by weight, 15 to 22% by weight, 15 to 21% by weight, 16 to 21% by weight, 17 to 21% by weight or 18 to 21% by weight, relative to the total weight of the composition for external application.

The concentration of propylene glycol diacetate may be, for example, 1 to 40% by weight, 2 to 40% by weight, 5 to 40% by weight, 10 to 35% by weight, 15 to 35% by weight, 20 to 35% by weight, 25 to 30% by weight, 5 to 15% by weight, 5 to 12% by weight or 8 to 10% by weight, relative to the total weight of the composition for external application.

The concentration of oleyl alcohol may be, for example, 0.4 to 8% by weight, 0.5 to 7% by weight, 1.0 to 7% by weight, 1.5 to 7% by weight, 1.8 to 7% by weight, 2.0 to 6.8% by weight, 2.2 to 6.6% by weight, 2.4 to 6.4% by weight, 2.5 to 6.2% by weight or 2.6 to 6.2% by weight, relative to the total weight of the composition for external application.

When the composition for external application of the present invention comprises additional organic solvent(s) with DMSO and oleyl alcohol, the concentration of the additional organic solvent(s) may be, for example, 5 to 90% by weight, 10 to 90% by weight, 15 to 90% by weight, 20 to 90% by weight, 25 to 90% by weight, 30 to 85% by weight, 40 to 85% by weight, 40 to 80% by weight, 5 to 60% by weight, 5 to 55% by weight, 5 to 50% by weight or 10 to 45% by weight, relative to the total weight of the composition for external application.

When the composition for external application of the present invention comprises DMSO and water, the concentration of water may be, for example, 0 to 3.0% by weight, 0 to 2.5% by weight, 0 to 2.0% by weight, 0 to 1.5% by weight, 0 to 1.0% by weight, 0 to 0.8% by weight, 0 to 0.6% by weight, 0 to 0.5% by weight, 0 to 0.4% by weight, 0 to 0.3% by weight, 0 to 0.2% by weight, 0 to 0.1% by weight, 0 to 0.08% by weight or 0 to 0.05% by weight, relative to the total weight of the composition for external application. When the concentration of water exceeds the upper value, the storage stability of baclofen may be reduced.

The composition for external application of the present invention may be used as a solution (a lotion). In addition, the composition for external application may be used in various types of external preparations such as gel agents, cream agents and patch preparations. When the composition for external application of the present invention is used as a solution, the solution may be applied to the skin, for example, by carrying an appropriate amount of the composition for external application of the present invention on a suitable carrier. Examples of the carrier include transdermal absorption preparation carrying members used in the delivery system for a transdermal absorption preparation described in WO 2016/136732 and WO 2017/082301. The contents of the references are hereby incorporated by this reference in its entirety. That is, the delivery system for a transdermal absorption preparation used herein may be the structure comprising:
(a) a solvent-impermeable first sheet;
(b) a solvent-impermeable second sheet attached to an upper surface of the first sheet, forming a non-sealing region and a sealing region surrounding the non-sealing region with the first sheet, and having a cutting part formed to annularly extend along an outer circumferential edge of the non-sealing region;
(c) a transdermal absorption preparation carrying member carrying the composition for external application of the present invention disposed between the first sheet and the second sheet in the non-sealing region and fixed to the second sheet inside the cutting part; and
(d) an adhesive third sheet attached in a removable manner to an upper surface of the second sheet.

When the composition for external application of the present invention is used as a patch preparation, the patch preparation may be prepared, for example, by mixing the composition for external application of the present invention with the well-known adhesive composition comprising a polymer such as a rubber polymer and an acrylic polymer as a base polymer to form an adhesive layer. The patch preparation of the present invention may be the structure comprising at least a support and an adhesive layer comprising an active ingredient laminated on one side of the support.

Examples of the rubber polymer include synthetic rubbers such as styrene-isoprene-styrene block copolymer (hereinafter, also referred to as "SIS"), styrene-butadiene-styrene block copolymer, styrene-ethylene-butadiene rubber-styrene block copolymer, styrene-butadiene rubber, polyisoprene, polyisobutylene and polybutene; and natural rubber, but are not limited thereto.

Examples of the acrylic polymer include acrylic acid-acrylic acid oxtyl ester copolymer, 2-ethylhexyl acrylate-vinylpyrrolidone copolymer, 2-ethylexyl acrylate-N-vinyl-2-pyrrolidone-dimethacrylic acid-1,6-hexaneglycol copolymer, acrylate-vinyl acetate copolymer and 2-ethylhexyl acrylate-2-hydroxyethyl acrylate-vinyl acetate copolymer, but are not limited thereto.

In the patch preparation of the present invention, the adhesive layer may further comprise other additive(s) such as a tackifier, a softener, a filler and an anti-oxidant.

Examples of the tackifier include rosin, rosin ester, hydrogenated rosin ester, terpene resin, terpene phenolic resin, C5-based petroleum resin, C5/C9-based petroleum resin, DCPD (dicyclopentadiene)-based petroleum resin, coumarone-indene resin and cycloaliphatic saturated hydrocarbon resin, but are not limited thereto. The tackifiers may be used alone. Also, two or more of the tackifiers may be used in combination.

Examples of the softener include petroleum softeners such as process oil and polybutene, fat oil-based softeners such as castor oil and coconut oil, purified lanolin, liquid paraffin and gelled hydrocarbon, but are not limited thereto. The softeners may be used alone. Also, two or more of the softeners may be used in combination.

Examples of the filler include kaolin, titanium oxide, talc, calcium carbonate, magnesium carbonate, silicate, silicic acid, aluminum hydrate, barium sulfate and calcium sulfate, but are not limited thereto. The filler can adjust the adhesive layer to an appropriate hardness when the adhesive layer becomes too flexible.

Examples of the anti-oxidant include dibutyl hydroxy toluene (BHT), butylated hydroxyanisole (BHA), propyl gallate, ascorbic acid, sodium sulfite, sodium hydrogen sulfite and sodium pyrosulfite, but are not limited thereto. The anti-oxidants may be used alone. Also, two or more of the anti-oxidants may be used in combination.

As the support in the patch preparation of the present invention, a drug-impermeable and stretchable or unstretchable support may be used. The support is not particularly limited thereto as long as it is usually used in the pharmaceutical field. Examples thereof include polyethylene, polypropylene, polybutadiene, ethylene-vinyl acetate copolymer, polyvinyl chloride, polyester (such as polyethylene terephthalate), film or sheet of synthetic resin such as nylon and polyurethan or laminated product thereof, porous material, foam, film with deposited aluminum, paper, woven cloth and non-woven cloth.

The patch preparation of the present invention may be prepared by any well-known methods such as the solvent method and the hot melt method.

The external preparation of the present invention may be packaged and stored by an appropriate packaging material. The packaging material may be a packaging material made of a material with low water permeability. Examples of the material with low water permeability include aluminum laminated film. The external preparation of the present invention is stored by the packaging material so that it does not come into contact with water, and thus can inhibit the generation of impurities and produce long-term storage stability of a drug.

The external preparation of the present invention may be used for the treatment of a disease associated with γ-aminobutyric acid (GABA) receptors such as cerebrovascular disorders, cerebral (child) paralysis, spastic spinal paralysis, vascular disorders of the spinal cord, cervical spondylosis, ossification of the posterior longitudinal ligament, multiple sclerosis, amyotrophic lateral sclerosis, spinocerebellar degeneration, posttraumatic sequelae (spinal cord injury, head injury) and postoperative sequelae (including brain and spinal cord tumors). The external preparation of the present invention may be attached or applied to the skin of a subject in need of treatment.

### EXAMPLES

Hereinafter, the present invention is described more specifically with reference to Examples. However, the present invention is not intended to be limited to them by any means.

### (Example 1)

Baclofen was dissolved in water, propylene glycol (PG), glycerin, acetic acid, lactic acid, 10% hydrochloric acid or 10% sodium hydroxide aqueous solution to prepare each solution comprising baclofen in an amount of 0.25% by weight. Each of the prepared solutions (10 g) was stored at 50°C, and the contexts of baclofen and ring products (intramolecular condensation products) thereof in each solution were measured after 1 and 2 weeks of the storage.

The results are shown in Table 1 below.

**[Table 1]**

| | | A-1 | A-2 | A-3 | A-4 | A-5 | A-6 | A-7 |
|---|---|---|---|---|---|---|---|---|
| | | Water | PG | Glycerin | Acetic acid | Lactic acid | 10% Hydrochloric acid | 10% NaOHaq |
| 50°C 1 week % (mg/ml) | Baclofen | 99.9 | 91.6 | 91 | 85.3 | 99.8 | 102.7 | 102.1 |
| | Ring products | 0.4 | 1.9 | 4 | 12.9 | 4.4 | 0 | 1.2 |
| 50°C 2 week % (mg/ml) | Baclofen | 98.6 | 73.1 | 93.1 | 70.5 | 98.3 | 100.3 | 100.3 |
| | Ring products | 0.9 | 10.2 | 6.3 | 23.4 | 1.3 | 0 | 0 |

The results showed that in the solutions of water, 10% hydrochloric acid, 10% sodium hydroxide aqueous solution and lactic acid, the generation of ring products of baclofen was inhibited.

On the other hand, in the solutions of propylene glycol and acetic acid, the content of baclofen was greatly reduced. In the solution of propylene glycol, degradation products of baclofen other than ring products were also generated in large amounts.

In addition, it was shown that baclofen was stable in water, whereas had low solubility in water (about 0.27%). The concentration of baclofen was insufficient to be used as an external preparation. Whereas, it was shown that baclofen had high solubility in 10% hydrochloric acid and lactic acid (10% hydrochloric acid: about 39%, lactic acid: about 33%), and was highly stable in the solutions thereof. Hence, the results suggested that the use of hydrochloric acid and lactic acid as a solubilizing agent could produce the enhanced stability of a preparation comprising baclofen.

### (Example 2)

Hydrochloric acid or lactic acid was used as the solubilizing agent to prepare each solution comprising baclofen. Each solution was dissolved in water, propylene glycol, NMP (N-methylpyrrolidone) or glycerin to assess the stability of baclofen in each of the prepared solutions at 50°C. The method of preparing each solution is as follows.

### [Solution comprising hydrochloric acid as solubilizing agent]

Baclofen was dissolved in 10% hydrochloric acid at the ratio of baclofen:10% hydrochloric acid = 1:2 (weight ratio) to prepare a solution of baclofen in hydrochloric acid. The solution of baclofen in hydrochloric acid (0.1 g) was then dissolved in each solvent (excluding water) (10 g) to prepare each sample.

### [Solution comprising lactic acid as solubilizing agent]

Baclofen was dissolved in lactic acid at the ratio of baclofen:lactic acid = 1:3 (weight ratio) to prepare a solution of baclofen in lactic acid. The solution of baclofen in lactic acid (0.1 g) was dissolved in each solvent (10 g) to prepare each sample.

The results are shown in Tables 2 and 3 below.

**[Table 2]**

| | | B-1 | B-2 | B-3 |
|---|---|---|---|---|
| | | NMP | Propylene glycol | Glycerin |
| 50°C 1 week % (mg/ml) | Baclofen | 99.7 | 62.3 | 82.3 |
| | Ring products | 0.8 | 0.2 | 0. 3 |
| 50°C 2 week % (mg/ml) | Baclofen | 97.1 | 41.9 | 68.5 |
| | Ring products | 2.6 | 2.3 | 3. 4 |

When hydrochloric acid was used as a solubilizing agent, baclofen dissolved in hydrochloric acid was unstable in propylene glycol and glycerin. Also, in the solution of baclofen in hydrochloric acid, products of baclofen other than ring products were generated.

**[Table 3]**

| | | C-1 | C-2 | C-3 | C-4 |
|---|---|---|---|---|---|
| | | Water | NMP | Propylene glycol | Glycerin |
| 50°C 1 week % (mg/ml) | Baclofen | 99.3 | 3.4 | 77 | 88.6 |
| | Ring products | 0 | 73.4 | 6 | 3.2 |
| 50°C 2 week % (mg/ml) | Baclofen | 98.8 | 0 | 55.1 | 86.4 |
| | Ring products | 2.3 | 91.2 | 35 | 17.6 |

When lactic acid was lactic acid as a solubilizing agent, baclofen dissolved in lactic acid disappeared in almost one week when dissolved in NMP and only ring products of baclofen were contained in NMP. Baclofen dissolved in lactic acid was unstable in propylene glycol, whereas was stable in glycerin and water.

### (Example 3)

### [Assessment of stability of baclofen hydrochloride solution]

Baclofen (10.0 g) was dissolved in 10% hydrochloric acid (20 g), the solvent in the prepared solution was frozen, and then the frozen product was dried in a lyophilizer overnight to give baclofen hydrochloride.

The resulting baclofen hydrochloride was dissolved in water, NMP (N-methylpyrrolidone), propylene glycol or glycerin to prepare each solution comprising baclofen hydrochloride in an amount of 0.25% by weight. Each of the prepared solutions, was stored at 50°C and the contexts of baclofen and ring products thereof in the solutions were measured after 1 and 2 weeks of the storage to assess the stability of baclofen.

The results are shown in Table 4 below.

**[Table 4]**

| | | D-1 | D-2 | D-3 | D-4 | |
|---|---|---|---|---|---|---|
| | | Water | NMP | Propylene glycol | Glycerin | |
| 50°C 1 week % (mg/ml) | Baclofen | 99.1 | 94.9 | 91.5 | 93.3 | |
| | Ring products | 0 | 0.8 | 0 | 0 | |
| 50°C 2 week % (mg/ml) | Baclofen | 98.6 | 92.8 | 87.9 -- | 89.2 | |
| | Ring products | 0.7 | 0 | 0 | 0 | |

Baclofen hydrochloride was stable even when dissolved in all of the solvents. In particular, the generation of ring products of baclofen was remarkably inhibited.

### (Example 4)

### [Assessment of stability and skin permeability of baclofen-containing transdermal preparation]

Each ingredient was weighed according to the composition (% by weight) in Tables 5 to 7 below and mixed. According to a conventional method, baclofen-containing transdermal preparations were prepared from each mixture. Each of the preparations was stored at 50°C and the contexts of baclofen and ring products thereof in the preparations were measured after 1 and 2 weeks of the storage to assess the stability of baclofen. In addition, the skin permeability of baclofen in each preparation was assessed according to *in vitro* test using the skin of a rat.

The results are shown in Tables 5 to 7 below.

**[Table 5]**

| | | E-1 | E-2 | E-3 | E-4 | E-5 |
|---|---|---|---|---|---|---|
| Baclofen | | 3.0 | 3.0 | 3.0 | 2.5 | 3.0 |
| 10% Hydrochloric acid | | | | | 5 | 5 |
| Lactic acid | | 7.5 | 7.5 | 7.5 | | |
| Phosphatidylcholine | | 0.25 | 0.25 | 0.25 | 0.25 | 0.25 |
| Propylene glycol | | 67 | 67 | 47 | 25.15 | 46.55 |
| Water | | 10 | 17.5 | 40 | | 5 |
| Glycerin | | 10 | 2.5 | | 66.4 | 40 |
| Oleyl alcohol | | 0.5 | 0.5 | 0.5 | 0.1 | 0.1 |
| Triethanolamine | | 1.75 | 1.75 | 1.75 | 0.6 | 0.6 |
| Total | | 100.0 | 100.0 | 100.0 | 100.0 | 100.5 |
| 50°C 1w % (mg/ml) | Baclofen | 87.2 | 81.6 | 92.4 | 95.7 | 97.4 |
| | Ring products | 4.0 | 3.49 | 1.8 | 0.33 | 0.43 |
| 50°C 2w % (mg/ml) | Baclofen | 72.2 | 71.8 | 85.3 | 95.9 | 95.5 |
| | Ring products | 6.78 | 6.12 | 3.34 | 0.74 | 0.86 |
| Skin permeation amount (µg/cm²) | 6hr | 144.5 | 208.0 | 14.7 | 0 | 0 |
| | 24hr | 2320 | 3797.5 | 265.35 | 19.7 | 38.56 |

The preparations comprising lactic acid showed higher skin permeability of baclofen as compared to the preparations comprising 10% hydrochloric acid. Whereas, the preparations comprising 10% hydrochloric acid showed more excellent effects on the preparation stability, particularly the inhibition of the generation of ring products of baclofen as compared to the preparations comprising lactic acid.

**[Table 6]**

| | | F-1 | F-2 | F-3 | F-4 | F-5 | F-6 |
|---|---|---|---|---|---|---|---|
| Baclofen hydrochloride | | 2.5 | 2.5 | 5.0 | 2.5 | 5.0 | 7.5 |
| Phosphatidylcholine | | 0.25 | 0.2 | 0.2 | 0.2 | 0.2 | 0.2 |
| Propylene glycol | | 96.25 | 94.55 | 92.05 | 91.8 | 89.3 | 88.7 |
| Oleyl alcohol | | 0.5 | 2.5 | 2.5 | 2.5 | 5.0 | 3.0 |
| Triethanolamine | | 0.5 | 0.25 | 0.25 | 0.5 | 0.5 | 0.6 |
| Total | | 100.0 | 100.0 | 100.0 | 100.0 | 100.0 | 100.0 |
| 50°C 1w % (mg/ml) | Baclofen | 88.8 | 89.9 | 94.2 | 94.4 | 93.4 | 92.6 |
| | Ring products | 6. 2 | 5.6 | 2.0 | 4.3 | 3.5 | 3.8 |
| 50°C 2w % (mg/ml) | Baclofen | 79.5 | 85.2 | 90.7 | 91.9 | 87.6 | 86.8 |
| | Ring products | 9.5 | 6.5 | 3.5 | 7.0 | 5.8 | 6.4 |
| Skin permeation amount 6hr (µg/cm²) | | 135.92 | 956 | 1923 | 1553 | 3082 | 5772 |

**[Table 7]**

| | | G-1 | G-2 | G-3 | G-4 | G-5 |
|---|---|---|---|---|---|---|
| Baclofen hydrochloride | | 2.5 | 2.5 | 2.5 | 2.5 | 2.5 |
| Phosphatidylcholine | | 0.2 | 0.2 | 0.2 | 0.2 | 0.2 |
| Propylene glycol | | 96.55 | 96.05 | 95.05 | 94.55 | 92.05 |
| Oleyl alcohol | | 0.5 | 1.0 | 2.0 | 2.5 | 5.0 |
| Triethanolamine | | 0.25 | 0.25 | 0.25 | 0.25 | 0.25 |
| Total | | 100 | 100 | 100 | 100 | 100 |
| 50°C 1w % (mg/ml) | Baclofen | 91.9 | 96.2 | 89.4 | 89.9 | 90.5 |
| | Ring products | 4.9 | 5.6 | 5.5 | 5.6 | 2.9 |
| 50°C 2w %(mg/ml) | Baclofen | 86.3 | 90.3 | 85.7 | 85.2 | - |
| | Ring products | 6.1 | 6.9 | 6.4 | 6.5 | - |
| Skin permeation amount 6hr (µg/cm²) | | 2.7 | 285.1 | 479.5 | 955.5 | 796.5 |

The preparations comprising baclofen hydrochloride instead of baclofen in the free form showed higher skin permeability as compared to the preparations comprising baclofen and 10% hydrochloric acid. In addition, the skin permeability was enhanced depending on the concentration of oleyl alcohol.

### (Example 5)

### [Assessment of stability and skin permeability of baclofen-containing transdermal preparation comprising DMSO]

Each ingredient was weighed according to the composition (% by weight) in Table 8 below and mixed. According to a conventional method, baclofen-containing transdermal preparations were prepared from each mixture. Each of the preparations was stored at 50°C and the contexts of baclofen and ring products thereof in the preparations were measured after 1 and 2 weeks of the storage to assess the stability of baclofen. In addition, the skin permeability of baclofen in each preparation was assessed according to *in vitro* test using the skin of a pig.

The results are shown in Table 8 below.

**[Table 8]**

| | | H-1 | H-2 | H-3 | H-4 | H-5 | H-6 | H-7 |
|---|---|---|---|---|---|---|---|---|
| Baclofen hydrochloride | | 7.5 | 7.5 | 7.5 | 7.5 | 7.5 | 7.5 | 7.5 |
| DMSO | | 89.5 | 45.0 | 44.0 | 92.5 | | 45 | 45 |
| Oleic acid | | | | 24.0 | | | | |
| Isostearic acid | | | | 21.5 | | | | |
| Diethyl sebacate | | | | | | | | 24 |
| PG-diAc | | | 44.5 | | | | | |
| Oleyl alcohol | | 3.0 | 3.0 | 3.0 | | | 3.0 | 3.3 |
| Propylene glycol | | | | | | 92.5 | | |
| DEGEE | | | | | | | 44.3 | 20 |
| Phosphatidylcholine | | | | | | | 0.2 | 0.2 |
| Total | | 100 | 100 | 100 | 100 | 100 | 100 | 100 |
| Accumulated skin permeation amount 24hr | | 1046.79 | 135.24 | 347.13 | 14.3 | | 2083.2 | 2250 |
| 50°C 1week % (mg/ml) | Baclofen | 101.0% | 99.7% | 99.3% | 99.7% | 60.5% | 96.2% | - |
| | Ring products | 0.3% | 0.3% | 0.8% | 0.3% | 0.7% | 0.7% | - |
| 50°C 2week % (mg/ml) | Baclofen | 99.9% | 99.5% | 97.2% | 9.5% | 79.10% | 91.9% | 95.3 |
| | Ring products | 0.7% | 0.8% | 1.6% | 0.5% | 0.40% | 1.3% | 1.3 |

| | | | | | | | | |
|---|---|---|---|---|---|---|---|---|
| DMSO: Dimethyl sulfoxide PG-diAc: Propylene glycol diacetate DEGEE: Diethylene glycol monoethyl ether | | | | | | | | |

The preparation comprising DMSO and substantially free of water showed extremely high storage stability.

### (Example 6)

### [Assessment of stability and skin permeability of baclofen-containing transdermal preparation comprising DMSO and C₁₆₋₂₀ fatty acid]

Each ingredient was weighed according to the composition (% by weight) in Table 9 below and mixed. According to a conventional method, baclofen-containing transdermal preparations were prepared from each mixture. Each of the preparations was stored at 80°C and the contexts of baclofen and ring products thereof in the preparations were measured after 2 days of the storage to assess the stability of baclofen. In addition, the skin permeability of baclofen in each preparation was assessed according to *in vitro* test using the skin of a pig.

The results are shown in Table 9 below.

**[Table 9]**

| | | J-1 | J-2 | J-3 | J-4 | J-5 | J-6 | J-7 | J-8 | J-9 | J-10 |
|---|---|---|---|---|---|---|---|---|---|---|---|
| Baclofen hydrochloride | | 30 | 30 | 30 | 30 | 30 | 30 | 30 | 22 | 22 | 30 |
| DMSO | | 59.5 | 35 | 58 | 54.5 | 40 | 45 | 30 | 27.5 | 27.5 | 40 |
| PG-diAc | | 10 | 10 | 10 | 10 | 10 | 10 | 10 | 29.5 | 24.5 | 10 |
| Oleic acid | | 0.5 | 1 | 2 | 5 | 0 | 0 | 0 | 0 | 0 | 1 |
| Isostearic acid | | 0 | 0 | 0 | 0 | 10 | 15 | 15 | 20 | 25 | 9 |
| Oleyl alcohol | | 0 | 0 | 0 | 0.5 | 0 | 0 | 0 | 0 | 0 | 0 |
| Ethyl acetate | | 0 | 24 | 0 | 0 | 10 | 0 | 15 | 0 | 0 | 10 |
| Oleth-20 | | 0 | 0 | 0 | 0 | 0 | 0 | 0 | 1 | 1 | 0 |
| Total | | 100 | 100 | 100 | 100 | 100 | 100 | 100 | 100 | 100 | 100 |
| Accumulated skin permeation amount 24hr | | 553.8 | 1959 | 6129 | 6029 | 1426 | 2366 | 1510 | 1679 | 1938 | 2275 |
| 80°C, 2days | Baclofen | 94.8% | 104.7% | 95.7% | 95.3% | 96.2% | 95.6% | 94.0% | 94.9% | 95.4% | 99.7% |
| | Ring products | 2.4 | 2.9% | 2.6% | 2.5% | 2.4% | 2.0% | | 2.5% | | 2.9% |

| | | | | | | | | | | | |
|---|---|---|---|---|---|---|---|---|---|---|---|
| MSO: Dimethyl sulfoxide PG-dAc: Propylene glycol diacetate leth-20: Polyoxyethylene(20)oleyl ether | | | | | | | | | | | |

### (Example 7)

### [Assessment of long-term stability of baclofen-containing transdermal preparation]

The baclofen-containing transdermal preparation of J-8 in Table 9 was prepared. The preparation was stored at 50°C, 40°C and 25°C for 3 months and the contexts of baclofen and ring products thereof in the preparations were measured after 1 month and 3 months of the storage to assess the long-term stability of baclofen.

The results are shown in Table 10 below.

**[Table 10]**

| | | | % (mg/ml) |
|---|---|---|---|
| 50°C | 1 month | Baclofen | 98.47 |
| | | Ring products | 0.89 |
| | 3 months | Baclofen | 94.43 |
| | | Ring products | 2.10 |
| 40°C | 1 month | Baclofen | 99.61 |
| | | Ring products | 0.18 |
| | 3 months | Baclofen | 98.48 |
| | | Ring products | 0.46 |
| 25°C | 1 month | Baclofen | 99.70 |
| | | Ring products | 0.00 |
| | 3 months | Baclofen | 99.47 |
| | | Ring products | 0.08 |

The preparations comprising DMSO and C₁₆₋₂₀ fatty acid showed high storage stability of baclofen over a long term.

### (Example 8)

### [Assessment of long-term stability of baclofen-containing transdermal preparation manufactured by machine]

A baclofen-containing transdermal preparation was manufactured by a machine according to the method of manufacturing a delivery system for transdermal absorption preparation described in WO 2016/136732 to assess the long-term stability of the preparation. In this working example, the baclofen-containing transdermal preparation (transdermal absorption preparation) of J-8 in Table 9 permeated a non-woven cloth (a transdermal absorption preparation carrying member) and was disposed between two aluminum laminated films (cover sheet and base sheet). Each of the resulting transdermal preparations was packaged in an aluminum bag and stored. The contexts of baclofen and ring products thereof in the preparations were then measured at the beginning (0 month) of the storage and after 1 month, 2 months and 3 months of the storage to assess the long-term stability of baclofen.

The results are shown in Table 11 below.

**[Table 11]**

| | | Beginning (0 month) | | 1 month | | 2 months | | 3 months | |
|---|---|---|---|---|---|---|---|---|---|
| | | Context (%) | Ring products (%) | Context (%) | Ring products (%) | Context (%) | Ring products (%) | Context (%) | Ring products (%) |
| 25°C | Mean value (n=3) | 101.6 | 0.00 | 101.0 | 0.07 | - | - | 99.8 | 0.13 |
| | SD | 4.5 | 0.00 | 1.7 | 0.02 | - | - | 6.7 | 0.01 |
| 40°C | Mean value (n=3) | 101.6 | 0.00 | 101.5 | 0.38 | 100.3 | 0.69 | 97.7 | 0.90 |
| | SD | 4.5 | 0.00 | 6.7 | 0.04 | 7.3 | 0.03 | 5.9 | 0.14 |

In the above table, the content (%) means the ratio of baclofen to the labeled amount thereof (about 44.4 mg). The machine-manufactured preparations comprising DMSO and a C₁₆₋₂₀ fatty acid also showed the high storage stability of baclofen over a long term.

### (Example 9)

### [Preparation of patch preparation]

The patch preparations were prepared with the compositions (% by weight) shown in Table 12 and Table 13 below. The preparations were prepared using ethyl acetate and heptane as a solvent according to the conventional solvent method.

**[Table 12]**

| | K-1 | K-2 | K-3 |
|---|---|---|---|
| Baclofen hydrochloride | 5 | 5 | 5 |
| DMSO | 20 | 20 | 20 |
| Oleic acid | 0 | 5.5 | 5.5 |
| Oleyl alcohol | 0 | 0 | 10 |
| Liquid paraffin | 19 | 13.5 | 3.5 |
| Light anhydrous silicic acid | 2 | 2 | 2 |
| Terpene resin | 38 | 38 | 38 |
| SIS5002 | 16 | 16 | 16 |
| Total | 100 | 100 | 100 |

**[Table 13]**

| | L-1 | L-2 | L-3 |
|---|---|---|---|
| Baclofen hydrochloride | 2.9 | 6.0 | 6.0 |
| Phosphatidylcholine | 0.1 | 0.1 | 0.1 |
| Propylene glycol | 8.8 | 9.0 | 9.2 |
| Triethanolamine | 0.1 | 0.3 | 0.3 |
| Oleyl alcohol | 2.9 | 3.0 | 3.1 |
| Light anhydrous silicic acid | 1.2 | 1.2 | 1.2 |
| Acrylic adhesive | 84.0 | 80.3 | 80.0 |
| Total | 100 | 100 | 100 |

## Claims

1. A composition for external application comprising baclofen hydrochloride.

2. The composition for external application according to claim 1, which further comprises DMSO.

3. The composition for external application according to claim 1 or 2, which further comprises one or more C₁₆₋₂₀ fatty acids.

4. The composition for external application according to claim 3, wherein the C₁₆₋₂₀ fatty acid is oleic acid, isostearic acid or a mixture thereof.

5. A patch preparation comprising a support and an adhesive layer laminated on one side of the support, wherein the adhesive layer comprises the composition for external application according to any one of claims 1 to 4.

6. A patch preparation comprising:
(a) a solvent-impermeable first sheet;
(b) a solvent-impermeable second sheet attached to an upper surface of the first sheet, forming a non-sealing region and a sealing region surrounding the non-sealing region with the first sheet, and having a cutting part formed to annularly extend along an outer circumferential edge of the non-sealing region;
(c) a transdermal absorption preparation carrying member carrying the composition for external application according to any one of claims 1 to 4 disposed between the first sheet and the second sheet in the non-sealing region and fixed to the second sheet inside the cutting part; and
(d) an adhesive third sheet attached in a removable manner to an upper surface of the second sheet.

7. A method of stabilizing a preparation comprising baclofen or a salt thereof, which comprises dissolving baclofen hydrochloride in an organic solvent and/or an inorganic solvent.

8. The method according to claim 7, wherein the organic solvent comprises DMSO.
